# EUROPÄISCHE PATENTSCHRIFT

(11) **EP 0 845 982 B1**
(45) Veröffentlichungstag und Bekanntmachung des Hinweises auf die Patenterteilung: **19.12.2001**
(21) Anmeldenummer: 96929292.9
(22) Anmeldetag: 21.08.1996
(51) Int. Cl.: A61K 9/20

(54) **VERWENDUNG VON LIPIDEN ALS HILFSMITTEL BEI DER HERSTELLUNG VON FESTEN ARZNEIFORMEN NACH DEM SCHMELZEXTRUSIONSVERFAHREN**
USE OF LIPIDS AS ADJUVENTS IN THE PRODUCTION OF SOLID MEDICINAL FORMS BY THE MELT EXTRUSION PROCESS
UTILISATION DE LIPIDES COMME ADJUVANTS DANS LA FABRICATION DE FORMES MEDICAMENTEUSES SOLIDES SELON LE PROCEDE D'EXTRUSION DE MATIERE FONDUE

(30) Priorität: 25.08.1995 DE 19531277
(43) Veröffentlichungstag der Anmeldung: 10.06.1998
(73) Patentinhaber: BASF AKTIENGESELLSCHAFT, 67056 Ludwigshafen (DE)
(72) Erfinder: ROSENBERG, Joerg, D-67158 Ellerstadt (DE); BREITENBACH, Jörg, D-68199 Mannheim (DE)
(86) Internationale Anmeldenummer: EP9603667
(87) Internationale Veröffentlichungsnummer: WO9707786

(56) Entgegenhaltungen:
- EP-A- 0 204 596
- WO-A-95/22319
- WO-A-96/14058
- DE-A- 4 413 350
- DE-A- 19 504 832

## Beschreibung

Die vorliegende Erfindung betrifft die Verwendung von Lipiden als Hilfsmittel bei der Herstellung von festen Arzneiformen nach dem Schmelzextrusionsverfahren.

Die Herstellung von festen Arzneiformen nach dem Schmelzextrusionsverfahren ist beispielsweise aus der US-A 4880585 bekannt, wobei die Herstellung der Arzneiform aus der noch plastischen, wirkstoffhaltigen Schmelze mit Hilfe eines Formkalanders gelingt, der direkt aus der Schmelze Tabletten formt.

Aus der Tablettierung von Granulaten ist bereits lange bekannt, daß in fast allen Fällen nur dann befriedigende Resultate erzielt werden, wenn den Formulierungen nach der Granulierung, aber vor der Tablettierung, eine geringe Menge eines "Schmiermittels" zugesetzt wird, das sich als feiner "Film" auf den Außenflächen der Granulatkörner niederschlägt und dadurch das "Kleben" der Granulate an den Tablettierstempeln verhindert. Der Einfluß dieser "Schmiermittel" ist sehr groß, obwohl nur sehr geringe Mengen den Granulaten zugeführt werden müssen (i.A. etwa 0,1 bis 1 Gew.-%; vergl. H. Sucker, P. Fuchs, P.Speiser: Pharmazeutische Technologie; Georg Thieme Verlag, Stuttgart (1991), Seite 259-260).

Bekannte Formentrennmittel für konventionelle Verfahren sind beispielsweise Fettsäureester oder Fettsäuresalze, wobei üblicherweise nur Magnesiumstearat eine praktische Anwendung findet. Das Magnesiumstearat wird üblicherweise fertigen wirkstoffhaltigen Granulaten vor der Tablettierung zugemischt.

Bei der Herstellung von Arzneiformen über Schmelzextrusionsverfahren in Verbindung mit einem Tablettierprozeß wie der Kalandrierung ist es entscheidend, daß die Schmelze keine Haftung auf den Oberflächen der Kalanderwalzen aufweist, da ansonsten eine Entformung nicht gelingt. Es ist weiterhin bei der Kalandrierung zu Tabletten zu beachten, daß als Folge des Preßvorgangs im Kalander Tabletten entstehen, die eine typische "Preßnaht" aufweisen, die sich an der Grenzfläche der sich nur oberflächlich berührenden Formwalzenpaare ausbildet. Diese Preßnaht muß im Anschluß an den Kalandriervorgang i.a. nach Abkühlen/ Aushärten der Schmelzetabletten mechanisch entfernt werden, um den Tabletten eine homogene Oberflächenstruktur zu geben. Das Gelingen dieses "Entgratungsverfahrens" ist entscheidend von der Konsistenz der erkalteten wirkstoffhaltigen Schmelze abhängig. Dies bedeutet, daß trotz eines guten Kalandrierverfahrens (keinerlei Kleben der Schmelze im Kalander) nicht notwendigerweise entgratbare Tabletten gebildet werden. Viele Polymere bilden thermoplastische Schmelzen auch in Gegenwart großer Mengen von Wirk- und/oder Hilfsstoffen, die nach dem Erkalten hochflexibel sind und ohne Bruch in weiten Bereichen biegsam sind. Die Entfernung der Preßnähte ist in diesen Fällen erheblich erschwert und oft völlig unmöglich, obwohl die Extrusion und auch die Kalandrierung problemlos verlaufen.

Entscheidend für die Verwendung einer Formulierung zur Herstellung von Tabletten über Schmelzextrusion/Kalandrierung ist somit neben einer guten Thermoplastizität der Gesamtmischung auch insbesondere die Möglichkeit zur Reduzierung der "Klebetendenz" beim Kalandriervorgang und die Kontrolle der Plastizität der erkalteten Schmelze, um die Entgratung der Tabletten sicherzustellen.

Thermoplastisch verarbeitbare Cellulosederivate wie z.B. Hydroxypropylcellulose sind als wasserlösliche Polymere gut zur Herstellung von Tabletten über lösungsmittelfreie Schmelzextrusion/ Kalandrierung geeignet. Durch Abmischen mit anderen Hilfsstoffen, z.B. den in Wasser quellbaren HPMC-Polymeren(Hydroxypropyl-Methyl-Cellulose) lassen sich die Auflösungszeiten solcher Tabletten im Magen-Darmtrakt gezielt steuern, wie beispielsweise aus der DE-A 4226753 bekannt ist.

Aus der EP-A 204 596 ist die Verwendung von Lipiden bei der Herstellung von Arzneimitteln durch Schmelzextrusion bekannt, wobei die Lipide in Mengen von 10 bis 40 Gew.-% eingesetzt werden. Die Lipide dienen dabei der Beeinflussung der Wirkstoffreisetzung.

Aus der WO 95/22319 ist ein Verfahren zur Herstellung von pharmazeutischen Formulierungen bekannt, wobei eine Mischung der Einsatzstoffe mit einem lipidischen Extrusionshilfsmittel durch Zugabe von Befeuchtungsmitteln wie beispielsweise Wasser vorgranuliert wird und das Granulat nach Extrusion durch eine Siebplatte getrocknet wird.

In der nachveröffentlichten WO 9528147 ist die Schmelzextrusion von Zusammensetzungen beschrieben, die neben einer lipidischen Komponente und einem Gelbildner mindestens ein wasserunlösliches Polymer enthalten.

Es wurde nun allerdings beobachtet, daß in vielen Fällen bei Verwendung von Hydroxypropylcellulosen als wasserlösliche, thermoplastische Polymerkomponente zwar gut extrudierbare Schmelzen erhalten wurden, die aber
a) in vielen Fällen extreme Klebetendenz beim Kalandrieren zeigten,
b) wegen zu großer Plastizität der erkalteten (wirkstoffhaltigen) Schmelzen im Fall der kalandrierten Tabletten eine Entfernung der "Preßnaht" nicht zuließen, und
c) wegen der hohen Klebetendenz der Schmelze erhebliche Probleme bei der Reinigung der Extruder verursachten.

Aufgabe der vorliegenden Erfindung war es Hilfsmittel zu finden, die vor allem bei der Verwendung von Hydroxypropylcellulosen als Matrixpolymere bei der Herstellung von Arzneiformen nach dem Schmelzextrusionsverfahren eine Lösung der oben genannten Probleme ermöglichen.

Demgemäß wurde die Verwendung von Lipiden als Formentrennmittel und Schmiermittel bei der Herstellung von Arzneiformen nach dem Schmelzextrusionsverfahren gefunden, wobei die Lipide in Mengen von 1 bis 5 Gew.-%, bezogen auf die Gesamtmenge der Arzneiformen, eingesetzt werden, als Matrixpolymere Hydroxypropylcellulosen und Polymere auf Basis von Vinylpyrrolidon eingesetzt werden und die Schmelze lösungsmittelfrei ist.

Als Lipide kommen erfindungsgemäß Mono-, Di- und Triglyceride von natürlich vorkommenden Fettsäuren in Betracht, beispielsweise Glycerolmonostearat, Glyceroldistearat, Glyceroltristearat, Glyceroltripalmitat, Glyceroltrimyristat, Glyceroltribehenat, Glycerolpalmitylstearylester oder Glyceridgemische wie sie in natürlichen Ölen vorkommen, vorzugsweise hydriertes Rizinusöl.

Weiterhin eignen sich auch Ceramide für diesen Zweck.

Bevorzugte Lipide sind vor allem Phospholipide, wobei Phosphoglyceride wie Lecithine besonders bevorzugt sind. Ganz besonders bevorzugt sind hydrierte Lecithine wie Soja- und Eilecithin.

Die Lipide können in Mengen von 1 bis 5 Gew.-%, bezogen auf die Gesamtmenge der wirkstoffhaltigen Zubereitungen, verwendet werden.

Die wirkstoffhaltigen Zubereitungen können als Matrixpolymere Hydroxypropylcellulosen und Polymere auf Basis von Vinylpyrrolidon enthalten, beispielsweise
- Polyvinylpyrrolidon,
- Copolymere aus N-Vinylpyrrolidon und Vinylacetat mit bis zu 50 Gew.-% Vinylacetat,

Als Wirkstoffe kommen alle Wirkstoffe in Betracht, die sich unter den Verarbeitungsbedingungen der Schmelzextrusion nicht zersetzen.

Die Menge der Wirkstoffkomponente in der Gesamtzubereitung kann je nach Wirksamkeit und Freisetzungsgeschwindigkeit in weiten Grenzen variieren. So kann der Wirkstoffgehalt im Bereich von 0,1 bis 90 Gew.-%, vorzugsweise von 0,5 bis 60 Gew.-%, bezogen auf die gesamte Zubereitung, liegen. Die einzige Bedingung ist, daß die Zubereitung noch thermoplastisch verarbeitbar ist.

Weiterhin können die Zubereitungen übliche pharmazeutische Hilfsstoffe wie Füllstoffe, Farbstoffe, Sprengmittel oder Stabilisatoren in üblichen Mengen enthalten.

Im übrigen erfolgt die Verarbeitung der Komponenten in an sich bekannter Weise in Extrudern, vorzugsweise in Ein- oder Doppelschneckenextrudern in einem Temperaturbereich zwischen 50 und 200°C. Die Formgebung der wirkstoffhaltigen, lösungsmittelfreien Polymerschmelze zu den erfindungsgemäßen Zubereitungen kann beispielsweise durch Kalandrierung des Extrudates sowie durch Zerkleinerung des Extrudates mit rotierenden Messern in volumengleiche - noch verformbare - Stücke mit erstarrter Oberfläche und anschließendes Verpressen zu Tabletten in den üblichen Tablettiermaschinen erfolgen.

Es ist möglich, die Hilfsstoffe in die Schmelze oder Lösung aus Wirkstoffen und Polymeren zu mischen. Ferner können die Hilfsstoffe zusammen mit dem Wirkstoff in die Polymerschmelze eingearbeitet werden. Außerdem können Gemische aus Hilfsstoffen, dem Wirkstoff und den Polymeren direkt verschmolzen werden. Im allgemeinen ist es üblich, eine physikalische Mischung aus Hilfsstoffen, Wirkstoffen und den Polymeren gemeinsam zu verschmelzen.

Überraschenderweise wurde gefunden, daß schon durch Zusatz kleiner Mengen an Lipiden das Kleben der wirkstoffhaltigen Schmelzen verhindert werden kann.

Schon ein Zusatz von 3 Gew.-% Lecithin verringert die Schmelzehaftung der extrudierten Masse derart, daß die Formulierungen ohne Einschränkungen kalandrierbar sind. Die Reinigung des Extruders von den ansonsten zähen, stark klebenden Schmelzeresten ist erheblich vereinfacht, da lecithinhaltige Rezepturen kaum noch Metallhaftung zeigen und "als Block" noch warm nach Beendigung der Extrusion von den noch betriebswarmen Extruder-Metallteilen entfernt werden können. Auch die Plastizität der erkalteten Schmelze wird positiv beeinflußt, so daß die Entgratung der Tabletten (Preßnaht-Entfernung) erheblich besser gelingt.

### Beispiele 1 bis 20

Alle Versuche wurden in einem Doppelschneckenextruder (ZSK-40-Extruder, Fa. Werner und Pfleiderer, Stuttgart) durchgeführt. Der Extruder beinhaltete 4 beheizbare Schüsse, auch der Extruderkopf und die Breitschlitzdüse waren separat beheizbar. Die eingestellten Temperaturen sind der Tabelle zu entnehmen. Die extrudierte Schmelze wurde in Form eines 12 bis 14 cm breiten Bandes über eine Breitschlitzdüse ausgetragen und anschließend in einem Formkalander, der aus einem gegenläufig rotierenden Formwalzenpaar (kühlbar) besteht, direkt zu Tabletten gepreßt. Die Tabletten besaßen eine längliche, stäbchenförmige Gestalt (Oblong-Tabletten ohne Steg). Die in der Tabelle aufgelisteten Rohstoffe wurden zuvor in einem Rhönradmischer gemischt und als Mischung in den Extruder über eine Dosierwaage mit Leistungen von 20 bis 30 kg/h eingetragen. Die Drehzahl der Extruder-Förderschnecke lag in allen Fällen bei 100 bis 150 U/min.

Zu Vergleichszwecken wurden die Formulierungen verarbeitet, bei denen kein Lipid zum Einsatz kam. In allen diesen Fällen konnte zwar extrudiert, nicht aber kalandriert werden, da die Entformung der Tabletten aus den Kalander-Formwalzenpaaren nicht gelang. Zudem war die Reinigung der produktberührenden Extruderteile in diesen Fällen erheblich schwieriger (stark an Metallflächen klebende Masse) als bei lipidhaltigen Formlierungen.

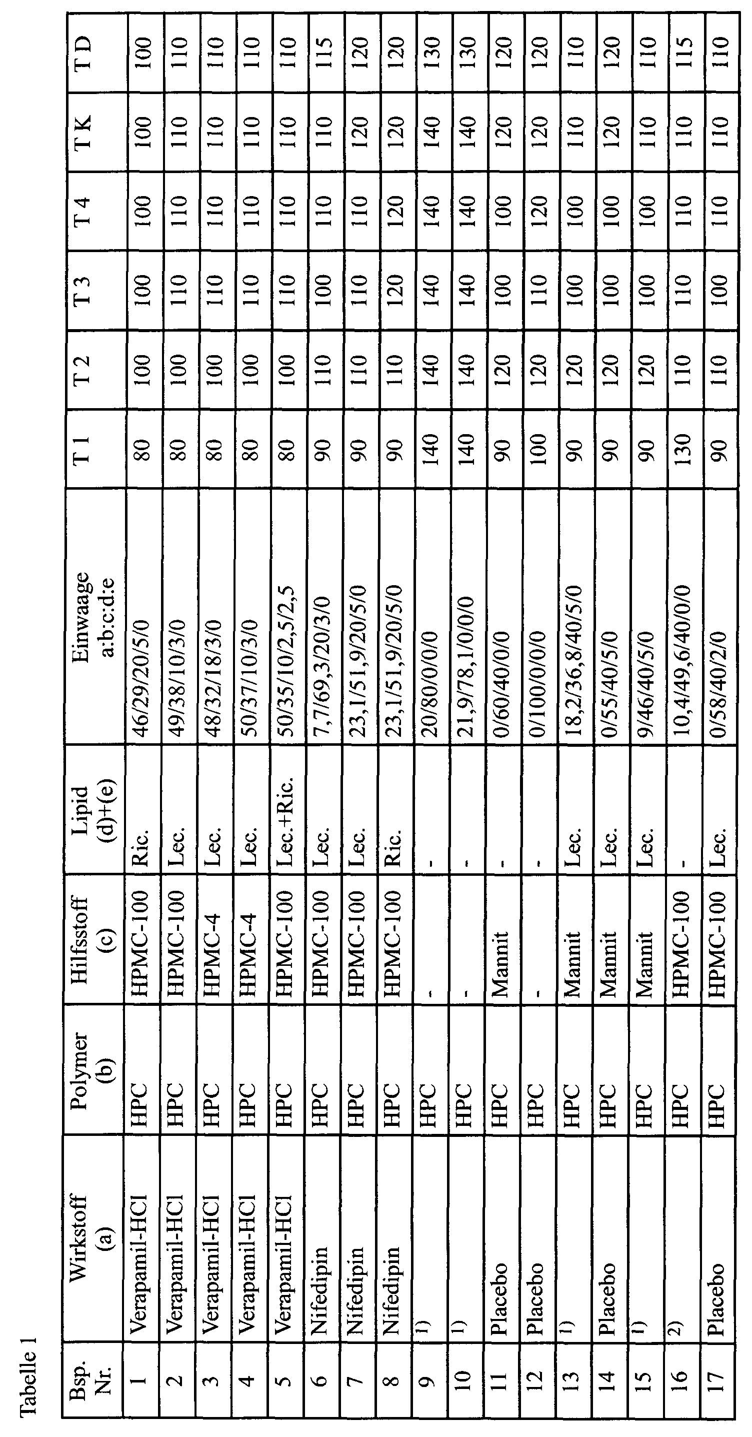

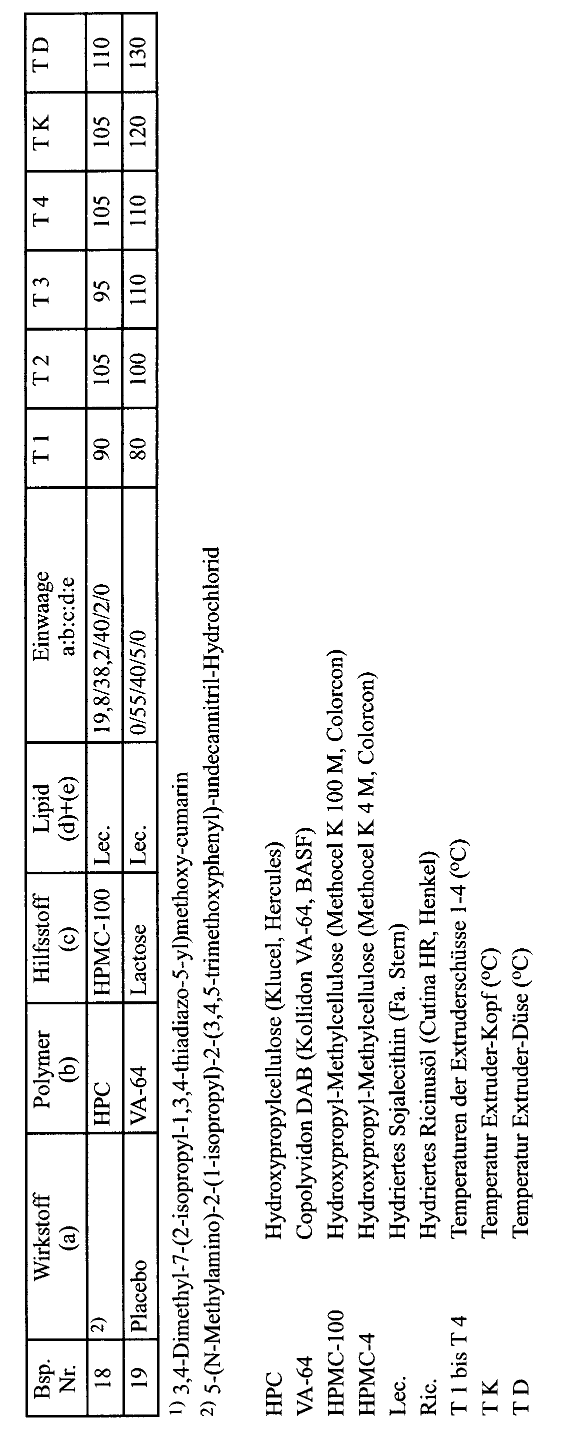

## Patentansprüche

1. Verwendung von Lipiden als Formentrennmittel und Schmiermittel bei der Herstellung von Arzneiformen nach dem Schmelzextrusionsverfahren, wobei die Lipide in Mengen von 1 bis 5 Gew.-%, bezogen auf die Gesamtmenge der Arzneiformen, eingesetzt werden, als Matrixpolymere Hydroxypropylcellulosen und Polymere auf Basis von Vinylpyrrolidon eingesetzt werden und die wirkstoffhaltige Polymerschmelze lösungsmittelfrei ist.

2. Verwendung nach Anspruch 1, wobei als Lipide Phosphoglyceride eingesetzt werden.

3. Verwendung nach Anspruch 2, wobei als Lipide Lecithine eingesetzt werden.

## Claims

1. The use of lipids as mold release agents and lubricants in the production of drug forms by the melt extension process, where the lipids are employed in amounts of from 1 to 5% by weight, based on the total amount of the drug forms, hydroxypropylcelluloses and vinylpyrrolidone-based polymers are employed as matrix polymers, and the active ingredient-containing polymer melt is free of solvents.

2. The use as claimed in claim 1, where phosphoglycerides are employed as lipids.

3. The use as claimed in claim 2, where lecithins are employed as lipids.

## Revendications

1. Utilisation de lipides en tant qu'agents de démoulage et lubrifiants à la préparation de médicaments par la technique d'extrusion à l'état fondu, dans laquelle les lipides sont mis en oeuvre en quantité de 1 à 5 % du poids total de la forme médicamenteuse, on utilise en tant que polymères de gangue des hydroxypropylcelluloses et des polymères à base de vinylpyrrolidone et la masse fondue de polymère contenant la substance active est exempte de solvant.

2. Utilisation selon la revendication 1, dans laquelle les lipides utilisés sont des phosphoglycérides.

3. Utilisation selon la revendication 2, dans laquelle les lipides utilisés sont des lécithines.
